# EUROPEAN PATENT APPLICATION

(11) **EP 4 538 254 A1**
(43) Date of publication of application: **16.04.2025**
(21) Application number: 23818906.2
(22) Date of filing: 17.05.2023
(51) Int. Cl.: C07C 17/10, C07C 19/01

(54) **CONTINUOUS PREPARATION METHOD FOR 1,1,1,2,3-PENTACHLOROPROPANE**

(30) Priority: 07.06.2022 CN 202210634874
(71) Applicant: Zhejiang Quhua Fluor-Chemistry Co Ltd, Quzhou, Zhejiang 324004 (CN)
(72) Inventor: ZHOU, Liyang, Quzhou, Zhejiang 324000 (CN); REN, Yawen, Quzhou, Zhejiang 324004 (CN); TONG, Chaoli, Quzhou, Zhejiang 324004 (CN); ZHANG, Yan, Quzhou, Zhejiang 324000 (CN); HONG, Jiangyong, Quzhou, Zhejiang 324000 (CN); YANG, Bo, Quzhou, Zhejiang 324000 (CN); ZHAO, Yang, Quzhou, Zhejiang 324000 (CN); YU, Huimei, Quzhou, Zhejiang 324004 (CN); XIONG, Xianyun, Quzhou, Zhejiang 324002 (CN)
(74) Representative: Becker, Eberhard
(86) International application number: PCT/CN2023/094743
(87) International publication number: WO 2023/236741

(57) **Abstract**

Disclosed is a continuous preparation method for 1,1,1,2,3-pentachloropropane. 1,1,1,3-tetrachloropropane and chlorine are continuously introduced into a reactor respectively and are subjected to a reaction under the action of a solid oxide catalyst to obtain the 1,1,1,2,3-pentachloropropane. The reaction is a gas-liquid-solid three-phase reaction, the 1,1,1,3-tetrachloropropane is introduced from the top of the reactor, and the chlorine is introduced from the top or the bottom of the reactor. The reaction condition is mild and the process is simple. In addition, the process also has the advantages of less three wastes, good conversion rate of 1,1,1,3-tetrachloropropane, high product selectivity, stable catalyst, and capability of achieving continuous production and the like, and has a wide industrial application prospect.

## Description

### TECHNICAL FIELD

The invention relates to a preparation method of a chlorinated hydrocarbon, and in particular to a continuous preparation method of 1, 1, 1, 2, 3-pentachloropropane.

### BACKGROUND

The new generation of environmentally friendly refrigerants 2, 3, 3, 3-tetrafluoropropene (HFO-1234yf), with an ozone depletion potential (ODP) of 0 and a greenhouse-warming potential (GWP) of only 4, is considered an ideal substitute for 1, 1, 1, 2-tetrafluoroethane (HFC-134a, ODP=0, GWP=1430).

In recent years, research on the preparation technology of 2,3,3,3-tetrafluoropropene has entered a period of rapid development, in which using 1,1,2,3-tetrachloropropene as a raw material to synthesize 2,3,3,3-tetrafluoropropene is an important way, and 1,1,2,3-tetrachloropropene can be synthesized by dehydrochlorination of 1,1,1,2,3-pentachloropropane. Therefore, how to efficiently synthesize 1,1,1,2,3-pentachloropropane is the key for preparing 2,3,3,3-tetrafluoropropene.

There are two main methods for preparing 1,1,1,2,3-pentachloropropane. One is an indirect method (a two-step method), where 1,1,1,3-tetrachloropropane is firstly dehydrochlorinated to obtain 1,1, 3-trichloropropene and/or 3,3,3-trichloropropene, which is then chlorinated to synthesize 1,1,1,2,3-pentachloropropane. Two is a direct method, where 1, 1, 1, 2, 3-pentachloropropane is prepared from direct chlorination of 1, 1, 1, 3-tetrachloropropane.

For example, CN108069817A discloses a method for high-selectivity and high-yield preparation of 1,1,1,2,3-pentachloropropane by a one-pot method, in which 1,1,1,3-tetrachloropropane is used as a raw material to prepare 1,1,3-trichloropropene by dehydrochlorination. When the content of 1, 1,3-trichloropropene in the system reaches 4% to 20%, chlorine is fed to carry out a reaction. After chlorine is introduced, the content of 1,1,3-trichloropropene in the reaction system is controlled at 1% to 6%. When the content of 1,1,1,2,3-pentachloropropane generated in the system reaches 90% to 99%, the reaction is terminated. The catalyst used is anhydrous ferric chloride or a composite catalyst composed of an iron powder and anhydrous ferric chloride, and the product yield is 95%. However, this invention has not yet mentioned the stability of the catalyst, and there are still many side reactions such as polymerization and isomerization. Meanwhile, the characteristic of this reaction is to pay close attention to the content of each substance in the reaction system, which imposes the inconvenience on the operation.

Another example is CN109232173A, which discloses a continuous preparation method of 1,1,1,2,3-pentachloropropane. In this invention, trichloropropene, used as a raw material, and chlorine are respectively fed into a microchannel preheater and preheated therein, and then the preheated materials are continuously fed into a microchannel reactor for reaction without light or a catalyst. The raw material conversion rate is 99% or above, and the product selectivity is 98% or above. Although the microchannel reactor can improve mass and heat transfer performance and shorten reaction time, solid materials cannot pass through the microchannels. If a large amount of solids are produced during the reaction, the microchannels are easily blocked. As a result, production cannot be carried out continuously. At the current technical level, it is difficult to achieve industrialization of microchannel reactors.

CN104130100A discloses methods of making chlorinated hydrocarbons. Under the action of dissolved ferric chloride, a liquid-phase chlorination is performed on 1,1,1,3-tetrachloropropane to prepare 1,1,1,2,3-pentachloropropane. The product selectivity is low, and the catalyst is unstable and can only work for five or six hours.

To sum up, the existing preparation technology mainly has the following shortcomings. In one aspect, the catalyst is unstable. Currently, anhydrous FeCl₃ Lewis acid catalyst is widely used. This catalyst is easily deactivated. Under a liquid phase condition, there is also the problem of difficult separation of a product from a catalyst, which is not conducive to industrial production. In another aspect, trichloropropene, a raw material/intermediate for preparing 1,1,1,2,3-pentachloropropane, is prone to self-polymerization during the reaction process, resulting in an increase in by-products and a decrease in product yield, making the subsequent system separation and purification complicated. Therefore, it is urgent to develop a safe and environmentally friendly preparation method of 1,1,1,2,3-pentachloropropane with simple process, good catalyst stability, and high production efficiency.

### SUMMARY

In response to the shortcomings in the prior art, the invention is intended to provide a safe and environmentally friendly continuous preparation method of 1,1,1,2,3-pentachloropropane with simple process, good catalyst stability, and high production efficiency.

In order to solve the above technical problems, the invention is implemented by the following technical solutions. A continuous preparation method of 1,1,1,2,3-pentachloropropane, comprising: continuously feeding 1,1,1,3-tetrachloropropane and chlorine into a reactor respectively, and carrying out a reaction in the presence of a solid oxide catalyst to obtain 1,1,1,2,3-pentachloropropane.

Preferably, the reaction is carried out at a temperature of 50 °C to 100 °C.

Preferably, the reaction is carried out under a pressure of 0.1 MPa to 0.5 MPa.

Preferably, a feed molar ratio of 1,1,1,3-tetrachloropropane to chlorine is 0.8-1.1:1.

Preferably, the solid oxide catalyst is at least one of magnesium oxide, iron oxide, zinc oxide, aluminum oxide, silicon oxide, and titanium oxide.

Preferably, the solid oxide catalyst is pretreated with chlorine before the reaction.

Preferably, the pretreatment is carried out at a temperature of 60 °C to 200 °C for 20 h to 40 h; the chlorine has a space velocity of 50 h⁻¹ to 100 h⁻¹.

Preferably, the reactor is a trickle bed reactor.

Preferably, the 1,1,1,3-tetrachloropropane and the chlorine flow in the reactor in a cocurrent or countercurrent manner.

In the preparation method of 1,1,1,2,3-pentachloropropane of the invention, 1,1,1,3-tetrachloropropane and chlorine are used as feedstocks to prepare 1,1,1,2,3-pentachloropropane by a one-step reaction in the presence of a solid oxide catalyst. In actual production, the obtained product can be subjected to conventional distillation and other purification operations in the art as needed to obtain a 1,1,1,2,3-pentachloropropane product with a higher purity. The preparation method of 1,1,1,2,3-pentachloropropane of the invention involves a gas-liquid-solid three-phase reaction, has advantages of simple process, mild reaction conditions, high conversion rate of 1,1,1,3-tetrachloropropane, high product selectivity, stable catalyst performance, continuous production and the like, and has broad industrial application prospects. The main chemical reactions in the invention are as follows:
Main reaction: 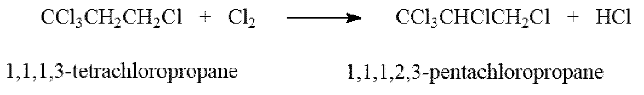
Side reaction:

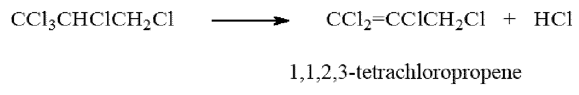

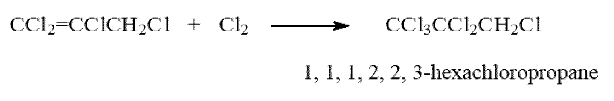

Compared with the prior art, the invention has the following advantages:
1. The process is simple. 1,1,1,2,3-pentachloropropane can be continuously prepared in a one-step reaction by using 1,1,1,3-tetrachloropropane and chlorine as feedstocks under the action of a solid oxide catalyst. The preparation process is significantly simplified, and since the oxide catalyst is a solid catalyst, which is easy to be separated from the product after the reaction is completed, the shortcomings in the prior art that the catalyst and the product are difficult to separate can be overcame.
2. The catalyst has good stability and high catalytic activity. The conversion rate of 1,1,1,3-tetrachloropropane is 90.3% or above and can reach up to 98.4%. The selectivity of 1, 1, 1, 2, 3-pentachloropropane is 93.1% or above, and can reach up to 98.2%. The activity of the catalyst does not decrease significantly after continuous operation for more than 360 h.
3. Easy to industrialize. The invention can continuously prepare 1,1,1,2,3-pentachloropropane by one-step reaction, and the catalyst has low preparation cost and is easy to obtain, which can be put into industrial mass production easily and has broad application prospects.
4. Low investment. The invention involves a one-step gas-liquid-solid three-phase reaction with mild reaction conditions. Compared with the two-step method, it significantly reduces the number of production devices and cost, and simplifies the operation and improves production safety in the meantime.

### BRIEF DESCRIPTION OF THE DRAWINGS

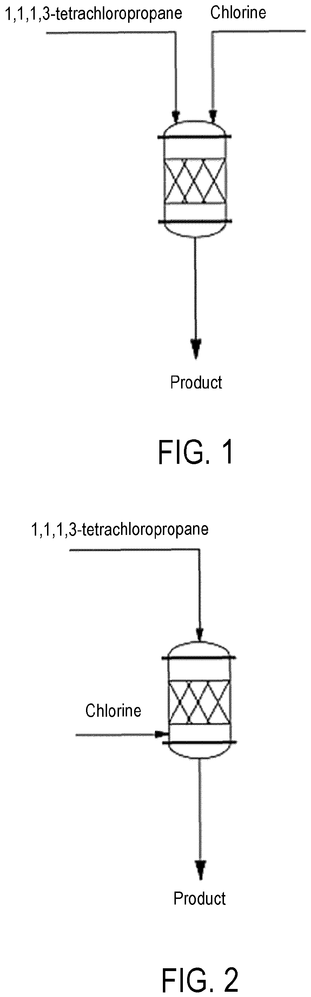

FIG. 1 is a schematic diagram of reaction forms in Examples 1-8 of the invention.
FIG. 2 is a schematic diagram of a reaction form in Example 9 of the invention.

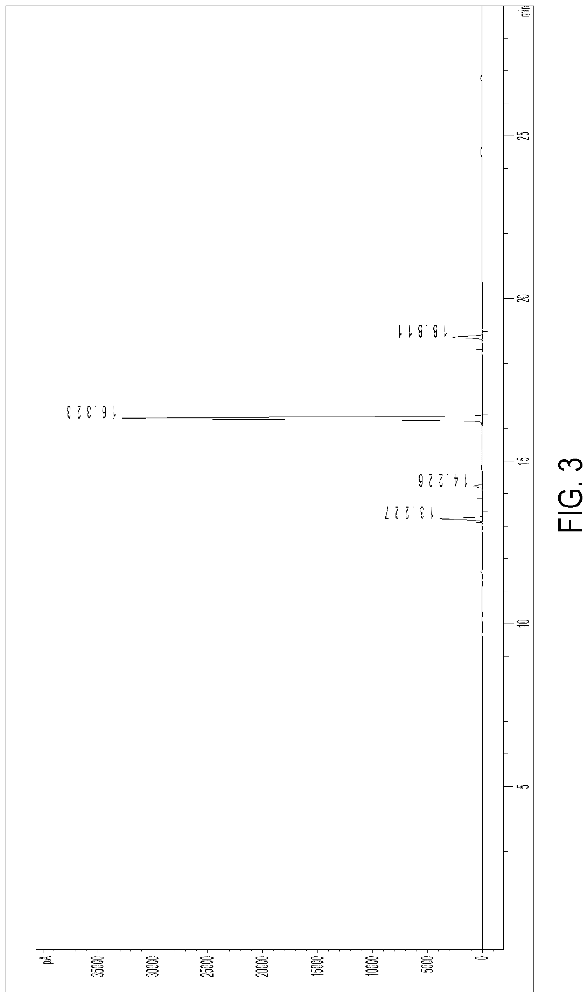

FIG. 3 is a chromatogram of a product obtained in Example 1 of the invention.

It can be seen from the figure that the chromatogram has a stable baseline, few impurity peaks, and no tailing or overlapping on the major peaks. The product can be well separated for quantitative analysis. The peak at 13.227 min represents 1,1,1,3-tetrachloropropane. The peak at 14.226 min represents 1, 1, 2, 3-tetrachloropropene. The peak at 16.323 min represents 1, 1, 1, 2, 3-pentachloropropane. The peak at 18.811 min represents 1, 1, 1, 2, 2, 3-hexachloropropane.

### DESCRIPTION OF THE EMBODIMENTS

The technical solution of the invention is clearly and completely described below in conjunction with embodiments. Apparently, embodiments described herein are merely part, not all of embodiments of the present invention. All other embodiments obtained by a person of ordinary skill in the art based on the embodiments of the invention without creative efforts shall fall within the scope of the invention.

### Example 1

120 mL of an alumina catalyst was fed into a trickle bed reactor (50 cm in length and 2.5 cm in diameter), and then chlorine was continuously fed from the top of the trickle bed reactor at a space velocity of 60 h⁻¹ to pretreat the catalyst. The trickle bed reactor was controlled at a temperature of 150 °C and under a pressure of 0.1 MPa and maintained for 20 h till the end of the pretreatment. After the pretreatment was finished, the catalyst was cooled to 70 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 1:1, and the pressure was 0.1 MPa. After the reaction was carried out for 24 h, the suctioned reaction product was collected. The reaction product was analyzed by gas chromatography. The conversion rate of 1,1,1,3-tetrachloropropane was 93.9%, and the selectivity of 1,1,1,2,3-pentachloropropane was 94.9%. After the reaction was carried out for 360 h, another sample was taken and analyzed and was found that the conversion rate of feedstocks and product selectivity did not decrease, indicating that the catalyst had good stability.

### Example 2

120 mL of an alumina catalyst was fed into a trickle bed reactor (50 cm in length and 2.5 cm in diameter), and then chlorine was continuously fed from the top of the trickle bed reactor at a space velocity of 50 h⁻¹ to pretreat the catalyst. The trickle bed reactor was controlled at a temperature of 120 °C and under a pressure of 0.1 MPa and maintained for 20 h till the end of the pretreatment. After the pretreatment was finished, the catalyst was cooled to 70 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 1:1, and the pressure was 0.1 MPa. After the reaction was carried out for 24 h, the suctioned reaction product was collected. The reaction product was analyzed by gas chromatography. The conversion rate of 1,1,1,3-tetrachloropropane was 91.8%, and the selectivity of 1,1,1,2,3-pentachloropropane was 97.2%. After the reaction was carried out for 360 h, another sample was taken and analyzed and was found that the conversion rate of feedstocks and product selectivity did not decrease, indicating that the catalyst had good stability.

### Example 3

120 mL of an alumina catalyst was fed into a trickle bed reactor (50 cm in length and 2.5 cm in diameter), and then chlorine was continuously fed from the top of the trickle bed reactor at a space velocity of 50 h⁻¹ to pretreat the catalyst. The trickle bed reactor was controlled at a temperature of 200 °C and under a pressure of 0.1 MPa and maintained for 25 h till the end of the pretreatment. After the pretreatment was finished, the catalyst was cooled to 70 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 0.8:1, and the pressure was 0.2 MPa. After the reaction was carried out for 24 h, the suctioned reaction product was collected. The reaction product was analyzed by gas chromatography. The conversion rate of 1,1,1,3-tetrachloropropane was 95.5%, and the selectivity of 1,1,1,2,3-pentachloropropane was 94.3%. After the reaction was carried out for 400 h, another sample was taken and analyzed and was found that the conversion rate of feedstocks and product selectivity did not decrease, indicating that the catalyst had good stability.

### Example 4

120 mL of an alumina catalyst was fed into a trickle bed reactor (50 cm in length and 2.5 cm in diameter), and then chlorine was continuously fed from the top of the trickle bed reactor at a space velocity of 50 h⁻¹ to pretreat the catalyst. The trickle bed reactor was controlled at a temperature of 60 °C and under a pressure of 0.1 MPa and maintained for 30 h till the end of the pretreatment. After the pretreatment was finished, the catalyst was cooled to 50 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 0.8:1, and the pressure was 0.5 MPa. After the reaction was carried out for 24 h, the suctioned reaction product was collected. The reaction product was analyzed by gas chromatography. The conversion rate of 1,1,1,3-tetrachloropropane was 92.3%, and the selectivity of 1,1,1,2,3-pentachloropropane was 98.2%. After the reaction was carried out for 480 h, another sample was taken and analyzed and was found that the conversion rate of feedstocks and product selectivity did not decrease, indicating that the catalyst had good stability.

### Example 5

120 mL of an alumina catalyst was fed into a trickle bed reactor (50 cm in length and 2.5 cm in diameter), and then chlorine was continuously fed from the top of the trickle bed reactor at a space velocity of 80 h⁻¹ to pretreat the catalyst. The trickle bed reactor was controlled at a temperature of 150 °C and under a pressure of 0.1 MPa and maintained for 20 h till the end of the pretreatment. After the pretreatment was finished, the catalyst was cooled to 80 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 1.1:1, and the pressure was 0.1 MPa. After the reaction was carried out for 24 h, the suctioned reaction product was collected. The reaction product was analyzed by gas chromatography. The conversion rate of 1,1,1,3-tetrachloropropane was 90.3%, and the selectivity of 1,1,1,2,3-pentachloropropane was 97.5%. After the reaction was carried out for 480 h, another sample was taken and analyzed and was found that the conversion rate of feedstocks and product selectivity did not decrease, indicating that the catalyst had good stability.

### Example 6

120 mL of an alumina catalyst was fed into a trickle bed reactor (50 cm in length and 2.5 cm in diameter), and then chlorine was continuously fed from the top of the trickle bed reactor at a space velocity of 100 h⁻¹ to pretreat the catalyst. The trickle bed reactor was controlled at a temperature of 150 °C and under a pressure of 0.1 MPa and maintained for 40 h till the end of the pretreatment. After the pretreatment was finished, the catalyst was cooled to 100 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 0.8:1, and the pressure was 0.1 MPa. After the reaction was carried out for 24 h, the suctioned reaction product was collected. The reaction product was analyzed by gas chromatography. The conversion rate of 1,1,1,3-tetrachloropropane was 98.4%, and the selectivity of 1,1,1,2,3-pentachloropropane was 93.1%. After the reaction was carried out for 480 h, another sample was taken and analyzed and was found that the conversion rate of feedstocks and product selectivity did not decrease, indicating that the catalyst had good stability.

### Example 7

120 mL of an alumina catalyst was fed into a trickle bed reactor (50 cm in length and 2.5 cm in diameter), and then chlorine was continuously fed from the top of the trickle bed reactor at a space velocity of 80 h⁻¹ to pretreat the catalyst. The trickle bed reactor was controlled at a temperature of 120 °C and under a pressure of 0.1 MPa and maintained for 20 h till the end of the pretreatment. After the pretreatment was finished, the catalyst was cooled to 70 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 0.8:1, and the pressure was 0.1 MPa. After the reaction was carried out for 24 h, the suctioned reaction product was collected. The reaction product was analyzed by gas chromatography. The conversion rate of 1,1,1,3-tetrachloropropane was 92.4%, and the selectivity of 1,1,1,2,3-pentachloropropane was 95.7%. After the reaction was carried out for 600 h, another sample was taken and analyzed and was found that the conversion rate of feedstocks and product selectivity did not decrease, indicating that the catalyst had good stability.

### Example 8

120 mL of an alumina catalyst was fed into a trickle bed reactor (50 cm in length and 2.5 cm in diameter), and then chlorine was continuously fed from the top of the trickle bed reactor at a space velocity of 50 h⁻¹ to pretreat the catalyst. The trickle bed reactor was controlled at a temperature of 120 °C and under a pressure of 0.1 MPa and maintained for 30 h till the end of the pretreatment. After the pretreatment was finished, the catalyst was cooled to 70 °C and at the same time, 1,1,1,3-tetrachloropropane was continuously fed at a flow rate of 60 g/h from the top of the trickle bed reactor to react with chlorine. During the reaction process, the molar ratio of 1,1,1,3-tetrachloropropane to chlorine was 0.8:1, and the pressure was 0.1 MPa. After the reaction was carried out for 24 h, the suctioned reaction product was collected. The reaction product was analyzed by gas chromatography. The conversion rate of 1,1,1,3-tetrachloropropane was 95.5%, and the selectivity of 1,1,1,2,3-pentachloropropane was 94.2%. After the reaction was carried out for 600 h, another sample was taken and analyzed and was found that the conversion rate of feedstocks and product selectivity did not decrease, indicating that the catalyst had good stability.

### Example 9

120 mL of an alumina catalyst was fed into a trickle bed reactor (50 cm in length and 2.5 cm in diameter), and then chlorine was continuously fed from the bottom of the trickle bed reactor at a space velocity of 50 h⁻¹ to pretreat the catalyst. The trickle bed reactor was controlled at a temperature of 150 °C and under a pressure of 0.1 MPa and maintained for 20 h till the end of the pretreatment. After the pretreatment was finished, the catalyst was cooled to 70 °C and 1,1,1,3-tetrachloropropane was continuously fed at a flow rate of 60 g/h from the top of the trickle bed reactor while chlorine was continuously fed from the bottom of the trickle bed reactor to perform a reaction. During the reaction process, the molar ratio of 1,1,1,3-tetrachloropropane to chlorine was 1:1, and the pressure was 0.1 MPa. After the reaction was carried out for 24 h, the suctioned reaction product was collected. The reaction product was analyzed by gas chromatography. The conversion rate of 1,1,1,3-tetrachloropropane was 95.2%, and the selectivity of 1,1,1,2,3-pentachloropropane was 93.8%. After the reaction was carried out for 720 h, another sample was taken and analyzed and was found that the conversion rate of feedstocks and product selectivity did not decrease, indicating that the catalyst had good stability.

The above are only the preferred embodiments of the invention. It should be pointed out that for those skilled in the art, without departing from the principles of the present invention, several improvements and modifications may also be made, and these improvements and modifications should also be regarded as falling within the scope of the invention.

## Claims

1. A continuous preparation method of 1,1,1,2,3-pentachloropropane, **characterized by** comprising: continuously feeding 1,1,1,3-tetrachloropropane and chlorine into a reactor, respectively; and carrying out a reaction in the presence of a solid oxide catalyst to obtain 1,1,1,2,3-pentachloropropane.

2. The continuous preparation method of 1,1,1,2,3-pentachloropropane according to Claim 1, **characterized in that**, the reaction is carried out at a temperature of 50 °C to 100 °C.

3. The continuous preparation method of 1,1,1,2,3-pentachloropropane according to Claim 1, **characterized in that**, the reaction is carried out under a pressure of 0.1 MPa to 0.5 MPa.

4. The continuous preparation method of 1,1,1,2,3-pentachloropropane according to Claim 1, **characterized in that**, a feed molar ratio of 1,1,1,3-tetrachloropropane to chlorine is 0.8-1.1:1.

5. The continuous preparation method of 1,1,1,2,3-pentachloropropane according to Claim 1, **characterized in that**, the solid oxide catalyst is at least one of magnesium oxide, iron oxide, zinc oxide, aluminum oxide, silicon oxide, and titanium oxide.

6. The continuous preparation method of 1,1,1,2,3-pentachloropropane according to Claim 1, **characterized in that**, a pretreatment is performed on the solid oxide catalyst with chlorine before the reaction.

7. The continuous preparation method of 1,1,1,2,3-pentachloropropane according to Claim 6, **characterized in that**, the pretreatment is carried out at a temperature of 60 °C to 200 °C for 20 h to 40 h, and the chlorine has a space velocity of 50 h⁻¹ to 100 h⁻¹.

8. The synthesis method according to Claim 1, **characterized in that**, the reactor is a trickle bed reactor.

9. The synthesis method according to Claim 1, **characterized in that**, the 1,1,1,3-tetrachloropropane and the chlorine flow in the reactor in a cocurrent or countercurrent manner.
